# EUROPEAN PATENT APPLICATION

(11) **EP 0 717 980 A2**
(43) Date of publication of application: **26.06.1996**
(21) Application number: 95119748.2
(22) Date of filing: 14.12.1995
(51) Int. Cl.: A61K 7/06

(54) **Use of guanidine-imidazole derivatives to assist hair growth stimulation**

(30) Priority: 23.12.1994 IT MI942645
(71) Applicant: ATELIER DYNAMIQUE S.r.l., I-20122 Milano (IT)
(72) Inventor: Razzano, Giovanni, I-22073 Fino Mornasco (Prov. of Como) (IT)
(74) Representative: Modiano, Guido, Dr.-Ing.

(57) **Abstract**

A new use of guanidine-imidazole derivatives that provides for their utilization in preparations of the dermatocosmetic type for topical application to the scalp to induce, maintain, or assist hair growth stimulation.

## Description

The present invention relates to the use of guanidine-imidazole derivatives to produce a composition for topical use to assist hair growth stimulation.

In particular, the present invention relates both to the use of guanidine-imidazole derivatives and of the related homologues to produce a hair cosmetic, and to a composition for topical application.

It is known that certain guanidine-imidazole compounds have a specific antagonistic activity with respect to H₂ histamine receptors. In particular, 1-cyano-2-methyl-3-[2-[ [ (5-methylimidazol-4-yl)methyl]thio]ethyl]-guanidine (cimetidine), its polymorphic forms, and its related homologues, are known to be strong inhibitors of H₂ histamine receptors and are used as active principles for curing gastric ulcer owing to this pharmacological activity.

The use of this molecule in the production of drugs for internal use, particularly in the preparation of pharmaceutical formulae for taking by mouth, has allowed to considerably reduce the incidence of acute attacks of peptic ulcer, allowing to avoid resorting to delicate surgery.

Cimetidine has been the subject of various patents (US 3 894 151, US 4 000 302, DE 2 320 131, GB 1 338 169), which also disclosed various homologue compounds thereof, different synthesis processes, and therapeutic treatment methods aiming at curing degenerative diseases of the gastric and esophageal mucosa, regions where the H₂ histamine receptors are mainly located. These receptors are responsible for the secretion of hydrochloric acid at the gastric level and therefore their inhibition considerably reduces the secretion of acid, which is the main responsible for the irritative and ulcerative phenomena affecting the gastric mucosa.

Various preparations are also known which are based on substances having a cosmetic and therapeutic activity and meant for topical use; they are recommended in the prevention and treatment of excessive hair loss.

In particular, it is known that minoxidil, which is used as an active principle for preparing pharmaceutical compositions for taking by mouth or parenterally, has shown, in the treatment of hypertension, by virtue of its peripheral vasodilatory activity, the ability to support the reproductive vitality of the scalp.

A principal aim of the present invention is to provide a composition for topical use that is particularly adapted in preventing and treating hair loss exceeding physiologic level.

An object is to provide a cosmetic hair product for topical use in the form of a solution which is simple and straightforward to use.

Another object is to provide a cosmetic hair product use whereof allows to give a better aesthetic appearance to hair and entails minimal risk of causing allergic reactions at the level of the scalp.

Another object is to provide a cosmetic hair product that is particularly adapted for treating women hair in the menopause period.

With this aim, these objects, and others in view, the invention provides for the use of a compound with formula I: 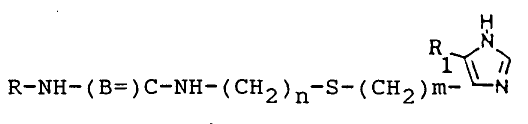
where R is
hydrogen, lower alkyl;
B is
NR₂;
R₂ is
hydrogen, cyano, nitro;
R₁ is
hydrogen, lower alkyl, halogen;
n and m are both whole numbers between 1 and 6, or of salts thereof; for the production of a composition for topical application, to assist hair growth stimulation; among these compounds, use of a compound with formula I is preferred,
where R is
methyl, ethyl, propyl
B is
NR₂;
R₂ is
cyano;
R₁ is
methyl, ethyl, propyl;
n and m are both 1, or of salts thereof;
among these compounds, the use of 1-cyano-2-methyl-3-[2-[[(5-methylimidazol-4-yl)methyl]thio]ethyl]-guanidine (cimetidine) or of salts thereof is particularly preferred.

The above mentioned compounds, and particularly cimetidine, therefore find, in the present invention, a new suggestion for use in the field of compositions for exclusively external use.

The present invention also relates to a composition for topical application for cosmetic treatment of hair, characterized in that it comprises a compound with formula I: 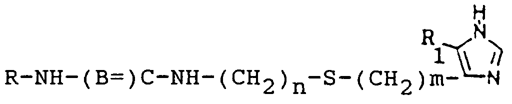
where R is
hydrogen, lower alkyl, and is preferably methyl, ethyl, propyl, and even more preferably methyl,
B is
NR₂;
R₂ is chosen in the group comprising
hydrogen, cyano, nitro, and is preferably cyano,
R₁ is chosen in the group comprising
hydrogen, lower alkyl, halogen, and is preferably methyl, ethyl, propyl, and even more preferably is methyl; and n and m are both whole numbers between 1 and 6 and are preferably 1, or of salts thereof and a topically acceptable carrier.

The new suggestion for use is matched by new formulae adapted to allow appropriate application of the active principle to the scalp.

The new use of the compounds disclosed herein, with particular reference to cimetidine, is mainly to be related to its regulatory mechanism affecting androgenic hormone equilibrium.

The hair bulb is, particularly in male hair, subjected to the aggressive action of hormone metabolites of the androgen type, which often cause an excessive secretion of sebum that suffocates the bulb and does not allow its correct transpiration.

The individual who is predisposed towards early hair loss is generally characterized by a hormone equilibrium in which dihydrotestosterone is predominant. This hormone, due to its hypocholesterolemic and hypoglycemic activity, produces, at the level of the hair bulb, a negative cellular energy balance, tending to remove energy, thus preventing a correct supply of nutrients. This process produces gradual thinning of the hair due to insufficient supply of nutrients, which are essential for its growth cycle.

Applicant has found that the administration at the topical level of the compounds with formula I, with particular reference to cimetidine, in the form of an appropriate formula, allows to act at the bulb level, restoring correct hormone metabolism. Cimetidine in fact plays a role in the process that regulates the reduction of testosterone to its derivative, dihydrotestosterone. This activity seems to be due to the ability to inhibit the 5-alpha reductase enzyme, which catalyzes the process for the reduction of testosterone to its dihydro derivative. Thanks to this property, the compounds with formula I, with particular reference to cimetidine, find a new indication in a field of application (that of compositions for external use) that is fully different from the field of the conventional use, using a mechanism of activity that is different from the one aimed at inhibiting the activity of H₂ histamine receptors.

The new use of the compounds with formula I and particularly of cimetidine preferably entails preparing a water-based composition in liquid form, more preferably in the form of a water solution and even more preferably in the form of a water/alcohol solution, which is particularly adapted for application at the level of the scalp.

This solution entails the use of the compounds with formula I and particularly of cimetidine, preferably at a concentration between 0.1% and 10%, more preferably between 0.3% and 5%, and even more preferably between 0.5% and 2% by weight.

The ethyl alcohol that is present in said water/alcohol solution is preferably included at a concentration between 1% and 50%, more preferably between 5% and 25%, and even more preferably between 10% and 15% by weight.

The water being used is preferably purified or demineralized water and is present in such amounts as to bring the final volume of the solution to 100.

The compounds with formula I have a synergistic effect in stimulating the growth or regrowth of hair when they are associated with nicotinic acid and salts thereof.

The use of a composition for topical application according to the present invention, which is particularly preferred, entails therefore the association of nicotinic acid or salts thereof, which is preferably included in concentrations between 0.1% and 5% and more preferably between 0.5% and 2%.

The composition according to the present invention furthermore provides for the use of preservatives, commonly used in the preparation of preparations for topical use, such as phenoxyethanol and imidazolidinylurea, preferably at concentrations between 0.10% and 1%, even more preferably between 0.40% and 0.50% by weight.

The following examples illustrate the present invention without limiting the scope thereof, as defined in the appended claims.

### EXAMPLE 1

| | |
|---|---|
| 1-cyano-2-methyl-3-[2-[[(5-methylimidazol-4-yl)methyl]thio]ethyl]-guanidine | 1% |
| Nicotinic acid | 1% |
| Preservatives (phenoxyethanol) | 0.40% |
| Ethyl alcohol | 12% |
| Purified water | q.s. to 100 |

### EXAMPLE 2

| | |
|---|---|
| 1-cyano-2-methyl-3-[2-[[(5-methylimidazol-4-yl)methyl]thio]ethyl]-guanidine | 2% |
| Nicotinic acid | 2% |
| Preservatives (phenoxyethanol, imidazolidinylurea) | 0.5% |
| Ethyl alcohol | 15% |
| Purified water | q.s. to 100 |

### EXAMPLE 3

| | |
|---|---|
| 1-cyano-2-methyl-3-[2-[[(5-methylimidazol-4-yl)methyl]thio]ethyl]-guanidine | 2% |
| Nicotinic acid | 1% |
| Preservatives (phenoxyethanol, imidazolidinylurea) | 0.5% |
| Ethyl alcohol | 15% |
| Purified water | q.s. to 100 |

### EXAMPLE 4

A composition for external use, to be applied to the scalp, has been produced, having the following composition:

| | |
|---|---|
| 1-cyano-2-methyl-3-[2-[[(5-methylimidazol-4-yl)methyl]thio]ethyl]-guanidine (cimetidine) | 1% by weight |
| Nicotinic acid | 0.5% by weight |
| Phenoxyethanol | 0.2% by weight |
| Imidazolidinylurea | 0.2% by weight |
| Ethyl alcohol | 15% by weight |
| Purified H₂O | 83.1% by weight |

### EXAMPLE 5

A cosmetic composition for improving the aesthetic appearance of hair, has been produced, having the following composition:

| | |
|---|---|
| Cimetidine | 0.5% by weight |
| Sodium nicotinate | 0.5% by weight |
| Phenoxyethanol | 0.2% by weight |
| Imidazolidinylurea | 0.2% by weight |
| Ethyl alcohol | 15% by weight |
| Demineralized H₂O | 83.6% by weight |

The preparation of the formulae for use according to the present invention preferably provides that the compounds with formula I and their salts be solubilized in the water/alcohol solutions mentioned above and that the synergistic agent, constituted by nicotinic acid, and the preservatives, be then added.

The compounds with formula I and their salts are preferably packaged in small bottles and even more preferably in glass vials or ampoules containing 2 to 5 cc of solution for optimum daily use of the product.

The preferred use of the compounds according to the present invention provides for the daily application of the dosage of the above mentioned vials preferably for a period of at least two months, preferably followed by a period of application every other day for an additional month.

The dosage of the vials must preferably be spread over the entire scalp, preferably by applying a gentle massage to facilitate local absorption of the active principles.

Use of the compounds according to the present invention preferably provides for at least two application cycles to be performed during the year.

Constant use of the compounds according to the present invention has proved itself particularly effective in stimulating hair growth in patients between 20 and 30 years of age, in whom the hair bulbs, despite being asphyxiated, are still capable of restoring good pilation activity.

Particularly encouraging results have furthermore been observed in the treatment of androgenic alopecia, against which the compounds according to the present invention have a high use-specificity.

Furthermore, the use of the compounds according to the present invention has been found to be particularly effective in the cosmetic treatment of the hair of menopausal women, which is often subjected to slower growth. Topical application of the composition according to the invention, in addition to assisting in the stimulation of hair growth, offers minimal allergological and toxicological risks in use. The use of the compounds according to the invention is advantageously aimed at producing a cosmetic product adapted to allow a cosmetic treatment to improve the consistency and external appearance of hair.

## Claims

1. Use of a compound with formula I: 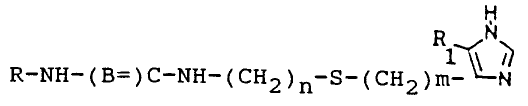 where R is
hydrogen, lower alkyl;
B is
NR₂;
R₂ is
hydrogen, cyano, nitro;
R₁ is
hydrogen, lower alkyl, halogen;
n and m are both whole numbers between 1 and 6;
or of salts thereof, for the production of a composition for topical application, to assist hair growth stimulation.

2. Use of a compound with formula I according to claim 1,
wherein R is
methyl, ethyl, propyl
B is
NR₂;
R₂ is
cyano;
R₁ is
methyl, ethyl, propyl;
n and m are both 1;
or of salts thereof.

3. Use of a compound according to any one of the preceding claims, wherein said compound is 1-cyano-2-methyl-3-[2-[[(5-methylimidazol-4-yl)methyl]thio]ethyl]- guanidine, or of salts thereof.

4. Use of a compound according to any one of the preceding claims, associated with nicotinic acid for topical application, to assist hair growth stimulation.

5. Use of a compound according to any one of the preceding claims, for producing a composition for topical application, in the form of a water/alcohol solution, to assist hair growth stimulation.

6. Use of a compound according to any one of the preceding claims, for producing a composition for topical application, suggested in the treatment of androgenic alopecia.

7. Use of a compound according to any one of the preceding claims, for producing a cosmetic product for assisting hair growth stimulation.

8. Use of a compound according to any one of the preceding claims, for producing a medicament for topical use to assist hair growth stimulation.

9. Composition for topical application, characterized in that it comprises a compound with formula I: 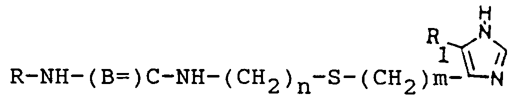 wherein R is
hydrogen, lower alkyl;
B is
NR₂;
R₂ is
hydrogen, cyano, nitro;
R₁ is
hydrogen, lower alkyl, halogen;
n and m are both whole numbers between 1 and 6,
or of salts thereof and a topically acceptable carrier.

10. Composition for topical application according to claim 9, characterized in that it comprises 1-cyano-2-methyl-3-[2-[[(5-methylimidazol-4-yl)methyl]thio]ethyl]-guanidine or salts thereof.

11. Composition according to any one of claims 9 and 10, characterized in that said compound with formula I is present at a concentration between 0.1 and 10% by weight.

12. Composition according to any one of claims 9 to 11, characterized in that it furthermore comprises nicotinic acid.

13. Composition according to any one of claims 9 to 12, characterized in that it is in the form of a water/alcohol solution.

14. Composition according to claim 13, characterized in that said solution has an alcohol concentration between 1 and 50% by weight.

15. Use of cimetidine as a hair growth stimulator.
